# EUROPEAN PATENT SPECIFICATION

(11) **EP 2 659 903 B1**
(45) Date of publication and mention of the grant of the patent: **14.09.2016**
(21) Application number: 11853373.6
(22) Date of filing: 04.11.2011
(51) Int. Cl.: A61K 38/13, A61K 47/30, A61K 47/44, A61P 27/02

(54) **NANOEMULSION-TYPE OPHTHALMIC COMPOSITION**
OPHTHALMISCHE NANOEMULSIONSZUSAMMENSETZUNG
COMPOSITION OPHTALMIQUE DE TYPE NANO-ÉMULSION

(30) Priority: 28.12.2010 KR 20100136667
(43) Date of publication of application: 06.11.2013
(73) Proprietor: Hanlim Pharmaceutical Co., Ltd., Gyeonggi-do 449-935 (KR)
(72) Inventor: LEE, Sung-Il, Seongnam-si Gyeonggi-do 463-781 (KR); YANG, Jae-Sik, Yongin-si Gyeonggi-do 446-908 (KR); LEE, Geun-Hyeog, Yongin-si Gyeonggi-do 446-722 (KR); CHOI, Byong-Sun, Seoul 150-096 (KR); RYU, Jong-Hyeon, Gwacheon-si Gyeonggi-do 427-040 (KR)
(74) Representative: Turner, Craig Robert
(86) International application number: PCT/KR2011/008359
(87) International publication number: WO 2012/091278

(56) References cited:
- KR-A- 20080 007 580
- KR-B1- 100 368 181
- KR-B1- 100 669 510
- US-A1- 2002 016 290
- US-A1- 2008 299 206
- US-A1- 2010 303 915
- FRONZA T ET AL: "Nanoemulsions as delivery systems for ophthalmic drugs", ACTA FARMACEUTICA BONAERENSE, COLEGIO DE FARMACEUTICOS DE LA PROVINCIA DE BUENOS AIRES, AR, vol. 23, no. 4, 1 January 2004 (2004-01-01), pages 558-566, XP008081023, ISSN: 0326-2383

## Description

### TECHNICAL FIELD

The present invention relates to an ophthalmic composition in a form of nanoemulsion. More specifically, the present invention relates to an ophthalmic composition designed to a nanoemulsion form, through self emulsification in an aqueous medium, using a certain oil and surfactant along with cyclosporin A as an active ingredient.

### BACKGROUND ART

Dry eye (i.e., keratoconjunctivitis sicca), some patients of which suffer from Sjogren's syndrome, is widespread especially in women after the menopause caused by hormonal changes according to loss of reproductive potential. Dry eye, which affects individuals in different severity, may bring about discomforts such as dryness, burning, and other symptoms in general; and damage of visual acuity in severe cases. Such a dry eye, which is caused by excessive evaporation of tears and insufficient production of tears, induces ocular surface problems, e.g., a series of symptoms such as foreign body sensation, burning sensation, etc.; and damage of visual acuity in severe cases, due to the damage of conjunctiva and cornea. Although dry eye may be derived from evaporation or insufficiency of tears, it is derived from overlap of the two causes in most cases.

Dry eye is defined as an abnormal state in the amount and quality of tears, regardless of keratoconjunctivitis sicca disorder (Nippon Ganka Kiyo. 1992 43: 1289-1293). According to the definition, disorders such as reduced shedding of tears, tear deficiency, dry eye syndrome, Sjogren's syndrome, keratoconjunctivitis sicca, Stevens-Johnson syndrome, ocular pemphigus, marginal blepharitis, failure of eyelid closure, sensory nerve injury, etc. are included in the category of dry eye. In many cases, dry eye results in insufficient tears in any one of the lipid layer, the aqueous layer and the mucin layer, thereby causing keratoconjunctivitis disorder.

As the most common method among various approaches for the treatment of dry eye, there is being mainly used an ocular instillation of artificial tears at the time when dry eye symptoms occur, so as to temporarily supplement and stabilize the tear film. These artificial tears, which contain viscoelastic materials such as methyl cellulose, chondroitin sulfate, and hyaluronic acid, are commonly applied to the eyes as a substitute for mucin. However, since the artificial tears are physically and physiologically different from mucin, the therapeutic effect thereof is limited.

Unlike medications for providing temporary relief to dry eye diseases, cyclosporin A is representatively used as a therapeutic agent for increasing tear secretion through stabilizing the epithelial cells and increasing goblet cells in the dry eyes. Because cyclosporin A inhibits the function of helper T cells through inhibiting the formation of interleukin-2 (one of the major inflammatory cytokines), which results in reducing lymphocytic infiltration of the lacrimal gland, thereby increasing tear production (J Korean Ophthalmol Soc 2009; 50(10): 1489-1494), the cyclosporin A is being used as an agent for treating dry eye diseases.

As an ophthalmic composition containing cyclosporin A, WO1995/31211 (Korean Patent Nos. 10-0368181 and 10-0450703) has disclosed a nonirritating ophthalmic composition which comprises cyclosporin A in admixture with castor oil, polysorbate 80, and a homopolymer of acrylic acid cross-linked with allyl ether of pentaerythritol (for example, Carbomer 1382, Pemulene™, etc.). The composition is prepared by mixing cyclosporin A with castor oil and polysorbate 80 to form an emulsion; and designed to have stability through using a viscoelastic material such as Carbomer 1382, Pemulene™, etc. Currently, the composition is being clinically used under the trade name Restasis™.

Another ophthalmic formulation containing cyclosporine is disclosed in US2008/0299206.

On the other hand, from the characteristics of an ophthalmic formulation, the formulation requires being prepared in a sterile form. However, the ophthalmic composition prepared from e.g., castor oil, polysorbate 80, and Pemulene^{™}, etc. according to WO1995/31211 is in a suspension-like emulsion form (in which the particles have the size ranging from 0.067 µm to 2.489 µm), which makes it impossible to carry out a conventional sterile filtration, i.e., sterile filtration with a 0.22 µm filter. Therefore, because the whole process for preparing the composition should be carried out in a closed system under sterile condition, there are disadvantages that it is difficult to perform the process at the manufacturing site in industrial scale and that the production cost thereof is very high.

In addition, in order to solve these problems, it can be considered to design an oleaginous solution form, through the use of an organic solvent such as ethanol. However, the use of an organic solvent in an ophthalmic formulation (not an oral formulation) brings about irritating problems, thereby lowering patients' drug compliance remarkably; and decreases stability of the formulation.

### DISCLOSURE

### Technical Problem

In order to address problems of the prior arts, the present disclosure relates to an ophthalmic composition to which a conventional sterile filtration with a 0.22 µm filter can be applied, wherein the composition does not require using an organic solvent and has excellent stability.

That is, it is an object of the present invention to provide a cyclosporin A-containing stable ophthalmic composition, to which a sterile filtration can be applied, thereby reducing the production cost and easily being prepared at the manufacturing site.

### Technical Solution

According to an aspect of the invention, there is provided an ophthalmic composition according to claim 1.

The present disclosure also relates to an ophthalmic composition in a form of nanoemulsion having a particle size of not more than 200 nm, which comprises cyclosporin A as an active ingredient; one or more oil selected from the group consisting of propylene glycol monocaprylate, propylene glycol dicaprylocaprate, propylene glycol laurate, medium chain (C₈ to C₁₀) triglycerides, glyceryl-1,3-dioleate, glyceryl monooleate, and glyceryl linoleate; and one or more surfactant selected from the group consisting of oleoyl macrogolglycerides, linoleoyl macrogolglycerides, caprylocaproyl polyoxylglycerides, polyoxyl 35 hydrogenated castor oil, polyoxyl 40 hydrogenated castor oil, a condensation product of ethylene oxide with 12-hydroxystearic acid, and polysorbate 80 in an aqueous medium.

In the ophthalmic composition of the present disclosure, the oil may be present in an amount ranging from 0.05 v/v% to 1.09 v/v%, based on the total composition; and the surfactant may be present in an amount ranging from 0.27 v/v% to 4.05 v/v%, based on the total composition. A volumetric ratio of the oil and the surfactant may be is in a range of 1:0.37 to 1:19. And also, the ophthalmic composition according to the present disclosure may further comprise one or more excipient selected from the group consisting of a buffering agent, an isotonic agent, and a pH control agent.

### ADVANTAGEOUS EFFECTS

The ophthalmic composition according to the present disclosure is a cyclosporin A-containing formulation designed to a nanoemulsion form through a self emulsification method (SMEDDS: Self Micro-Emulsion Drug Delivery System). The ophthalmic composition according to the present disclosure is thermodynamically stable; and has a particle size of not more than 200 nm, which makes it possible to carry out sterile filtration with a 0.22 µm filter. Therefore, the ophthalmic composition according to the present disclosure has advantages that the production cost thereof can be reduced and that the production thereof is easily adapted at the manufacturing site. And also, because the ophthalmic composition according to the present disclosure is thermodynamically stable, excellent stability is obtainable even without using a viscoelastic polymer such as Carbomer 1382, Pemulene™, etc. This property is very advantageous, when considering that, from the characteristics of an ophthalmic formulation, it is desired in terms of safety that excipients in the formulation should be minimized. Therefore, the ophthalmic composition of the present disclosure can be usefully used, e.g., in a form of eye drop for treating dry eye.

### DESCRIPTION OF DRAWINGS

FIG. 1 is the results obtained by measuring particle size distribution of the eye drop solution prepared according to the present disclosure (the eye drop solution of Example 1).
FIG. 2 is the results obtained by measuring particle size distribution of Restasis™ (Allergan Incorporated).

### BEST MODE

The present disclosure provides an ophthalmic composition designed to a nanoemulsion form, i.e., an ophthalmic composition in a form of nanoemulsion having a particle size of not more than 200 nm (preferably 10 to 200 nm, more preferably 10 to 100 nm), through self emulsification in an aqueous medium, using a certain oil and surfactant along with cyclosporin A as an active ingredient.

The ophthalmic composition according to the present disclosure has a particle size of not more than 200 nm, which makes it possible to carry out sterile filtration with a 0.22 µm filter. Therefore, the production cost thereof can be reduced; and the production thereof can be easily adapted at the manufacturing site. And also, because the ophthalmic composition according to the present disclosure is thermodynamically stable, excellent stability is obtainable even without using a viscoelastic polymer such as Carbomer 1382, Pemulene™, etc. The ophthalmic composition of the present disclosure has very advantageous properties, when considering the well-known fact that, from the characteristics of an ophthalmic formulation, it is desired in terms of safety that excipients in the formulation should be minimized.

The cyclosporin A contained as an active ingredient may be present in a therapeutically effective amount, which may be appropriately determined by a skilled person in the art. For example, cyclosporin A may be present in an amount raging from 0.05 to 0.20 w/v%, preferably about 0.05 w/v%, based on the total composition. Preferably, the cyclosporin A has an average particle diameter of 1 to 100 µm and D₅₀ (average particle size) of 10 to 20 µm.

The oil consists of propylene glycol dicaprylocaprate [for example, Labrafac™ PG, etc.], and medium chain (C8 to C10) triglycerides [for example, Labrafac Lipophile™ WL 1349, etc.]. The oil may be present in an amount ranging from 0.05 v/v% to 1.09 v/v%, preferably 0.05 v/v% to 0.68 v/v%, based on the total composition.

The surfactant is a polyoxyl 35 hydrogenated castor oil [for example, Cremophor^{™} EL (BASF, Germany), Cremophor^{™} ELP (BASF, Germany), etc.]. The surfactant may be present in an amount ranging from 0.27 v/v% to 4.05 v/v%, preferably 0.27 v/v% to 0.96 v/v%, based on the total composition.

And also, the volumetric ratio of the oil and the surfactant is in a range of 1:0.37 to 1:19, preferably 1:0.67 to 1:5.

The ophthalmic composition according to the present disclosure may contain excipients conventionally used in an ophthalmic formulation, for example, excipients such as a buffering agent, an isotonic agent, and a pH control agent.

As a buffering agent, sodium hydrogen phosphate (or its hydrate), sodium dihydrogen phosphate (or its hydrate), are used. A skilled person in the art may appropriately select an amount of the buffering agent. For example, sodium hydrogen phosphate (or its hydrate) and sodium dihydrogen phosphate (or its hydrate) may be used in an amount of 0.80 w/v% to 3.00 w/v%, respectively.

As an isotonic agent, sodium chloride, potassium chloride, boric acid, borax, etc. may be used, but not limited thereto. A skilled person in the art may appropriately select an amount of the isotonic agent. For example, when sodium chloride is used, sodium chloride may be used in an amount of 0.75 w/v% to 0.95 w/v%. When sodium chloride, boric acid, and borax are used, these may be used in an amount of 0.05 w/v% to 1.00 w/v%, respectively.

And also, the pH control agent may be added for adjusting the pH of the resulting ophthalmic composition to an appropriate range, for example, pH 5.0 to 8.0, preferably about pH 7.0. Typically, hydrochloric acid or sodium hydroxide may be used. The pH control agent may be used in an amount required for obtaining an appropriate pH range, without special limitation.

In the ophthalmic composition of the present disclosure, sterile distilled water, water for injection, etc. suitable for preparing an ophthalmic formulation may be used as an aqueous medium. The ophthalmic composition according to the present disclosure may be used in a conventional eye drop form.

The present disclosure also relates to an ophthalmic composition in a form of nanoemulsion, the eye irritancy of which is minimized. In accordance with the present disclosure, there is disclosed an ophthalmic composition in a form of nanoemulsion having a particle size of not more than 200 nm, which comprises cyclosporin; propylene glycol dicaprylocaprate and medium chain (C₈ to C₁₀) triglycerides as an oil; and polyoxyl 35 hydrogenated castor oil as a surfactant in an aqueous medium. In this example, the oil may be present in an amount ranging from 0.05 v/v% to 0.80 v/v%, preferably from 0.05 v/v% to 0.37 v/v%, based on the total composition; the surfactant may be present in an amount ranging from 0.63 v/v% to 1.32 v/v%, preferably from 1.00 v/v% to 1.15 v/v%, based on the total composition. And also, in a preferable example, there is provided an ophthalmic composition in a form of nanoemulsion having a particle size of not more than 200 nm, which comprises 0.05 w/v% of cyclosporin; 0.05 v/v% of propylene glycol dicaprylocaprate; 0.37 v/v% of medium chain (C₈ to C₁₀) triglycerides; and 1.15 v/v% of polyoxyl 35 hydrogenated castor oil in an aqueous medium. In said example and the preferable example, the ophthalmic composition may further comprise sodium hydrogen phosphate or its hydrate, sodium dihydrogen phosphate or its hydrate, or a mixture thereof as a buffering agent, preferably, 2.72 w/v% of sodium hydrogen phosphate or its hydrate; and 0.84 w/v% of sodium dihydrogen phosphate or its hydrate. In an especially preferable example, there is provided an ophthalmic composition in a form of nanoemulsion having a particle size of not more than 200 nm, which comprises 0.05 w/v% of cyclosporin; 0.05 v/v% of propylene glycol dicaprylocaprate; 0.37 v/v% of medium chain (C₈ to C₁₀) triglycerides; 1.15 v/v% of polyoxyl 35 hydrogenated castor oil; 2.72 w/v% of sodium hydrogen phosphate (or its hydrate); 0.84 w/v% of sodium dihydrogen phosphate (or its hydrate) in an aqueous medium.

The present disclosure will be described in further detail with reference to the following examples. These examples are for illustrative purposes only and are not intended to limit the scope of the present invention.

### Example

Examples 1 to 42 described below are not according to the present invention. Examples 43 to 48 are according to the present invention.

Eye drop solutions in the nanoemulsion form were prepared according to the components and amounts shown in Tables 1 to 8. Using a stirrer, the surfactant was thoroughly mixed with the oil; and then cyclosporin A was completely dissolved therein. Sterile distilled water (90 ml) was added to the mixture, which was then thoroughly mixed. Tonicity was adjusted to 0.9 with the buffering agent and/or the isotonic agent; and then the pH was adjusted to about 7.0 with the pH control agent. By adding sterile distilled water to the resulting solution, the total volume was adjusted to 100 ml. The resulting solution was filtered through a 0.22 µm filter and then filled in an eye drop container.

**<Table 1>**

| | Component | Example | | | | | |
|---|---|---|---|---|---|---|---|
| | | 1 | 2 | 3 | 4 | 5 | 6 |
| Active ingredient | Cyclosporin (w/v%) | 0.05 | 0.05 | 0.05 | 0.05 | 0.05 | 0.05 |
| Oil | Capryol PGMC (v/v%) | 0.28 | 0.47 | 0.28 | 0.47 | 0.46 | 0.46 |
| | Plurol oleique CC497 (v/v%) | 0.06 | 0.06 | - | - | - | - |
| Surfactant | Labrafil M 2125CS (v/v%) | - | - | 0.06 | - | - | - |
| | Labrasol (v/v%) | - | - | - | 0.06 | 0.12 | 0.23 |
| | Cremophor EL (v/v%) | 0.66 | 0.47 | 0.66 | 0.47 | 0.42 | 0.31 |
| Buffering agent | Sodium hydrogen phosphate hydrate (w/v%) | 2.72 | - | 0.91 | - | 2.72 | 2.72 |
| | Sodium dihydrogen phosphate hydrate (w/v%) | 0.84 | - | 0.96 | - | 0.84 | 0.84 |
| Isotonic agent | Sodium chloride (w/v%) | - | 0.88 | - | 0.70 | - | - |
| | Potassium chloride (w/v%) | - | - | - | 0.15 | - | - |
| | Boric acid (w/v%) | - | - | 0.27 | - | - | - |
| | Borax (w/v%) | - | - | 0.10 | - | - | - |
| pH control agent | HCl/NaOH | - | q.s. | q.s. | q.s. | - | - |
| | Distilled water (ml) | 100 | 100 | 100 | 100 | 100 | 100 |

**<Table 2>**

| | Component | Example | | | | | |
|---|---|---|---|---|---|---|---|
| | | 7 | 8 | 9 | 10 | 11 | 12 |
| Active ingredient | Cyclosporin (w/v%) | 0.05 | 0.05 | 0.05 | 0.05 | 0.05 | 0.05 |
| Oil | Capryol PGMC (v/v%) | 0.05 | - | 0.08 | 0.07 | 0.14 | 0.07 |
| | Labrafac Lipophile WL 1349 (v/v%) | 0.50 | 0.50 | 1.00 | 0.30 | 0.54 | 0.15 |
| Surfactant | Labrasol (v/v%) | - | 0.05 | - | - | - | - |
| | Cremophor ELP (v/v%) | 0.63 | 0.63 | 1.00 | 0.63 | 1.32 | 0.85 |
| Buffering agent | Sodium hydrogen phosphate hydrate (w/v%) | 2.72 | 2.72 | 2.72 | 2.72 | 2.72 | 2.72 |
| | Sodium dihydrogen phosphate hydrate (w/v%) | 0.84 | 0.84 | 0.84 | 0.84 | 0.84 | 0.84 |
| | Distilled water (ml) | 100 | 100 | 100 | 100 | 100 | 100 |

**<Table 3>**

| | Component | Example | | | | | |
|---|---|---|---|---|---|---|---|
| | | 13 | 14 | 15 | 16 | 17 | 18 |
| Active ingredient | Cyclosporine (w/v%) | 0.10 | 0.10 | 0.05 | 0.05 | 0.20 | 0.20 |
| Oil | Capryol PGMC (v/v%) | 0.10 | 0.40 | - | - | - | - |
| | Capryol 90 (v/v%) | - | - | 0.10 | 0.70 | 0.10 | 0.70 |
| | Labrafac PG (v/v%) | 0.20 | - | - | - | - | - |
| | Plurol oleique CC497 (v/v%) | - | 0.20 | - | - | - | - |
| | Maisine 35-1 (v/v%) | - | - | 0.09 | 0.03 | 0.09 | 0.03 |
| Surfactant | Labrafil M 1944CS (v/v%) | 0.20 | - | - | - | - | - |
| | Cremophor ELP (v/v%) | 0.50 | - | 0.81 | 0.27 | 0.81 | 0.27 |
| | Cremophor RH 40 (v/v%) | - | 0.40 | - | - | - | - |
| Buffering agent | Sodium hydrogen phosphate hydrate (w/v%) | 2.72 | 2.72 | 2.72 | 2.72 | 2.72 | 2.72 |
| | Sodium dihydrogen phosphate hydrate (w/v%) | 0.84 | 0.84 | 0.84 | 0.84 | 0.84 | 0.84 |
| | Distilled water(ml) | 100 | 100 | 100 | 100 | 100 | 100 |

**<Table 4>**

| | Component | Example | | | | | |
|---|---|---|---|---|---|---|---|
| | | 19 | 20 | 21 | 22 | 23 | 24 |
| Active ingredient | Cyclosporine (w/v%) | 0.05 | 0.05 | 0.05 | 0.05 | 0.05 | 0.05 |
| Oil | Capryol 90 (v/v%) | 0.28 | 0.47 | 0.10 | 0.70 | - | - |
| | Lauroglycol FCC (v/v%) | - | - | - | - | 0.10 | 0.70 |
| | Peceol (v/v%) | 0.06 | 0.06 | - | - | - | - |
| | Maisine 35-1 (v/v%) | - | - | 0.09 | 0.03 | 0.09 | 0.03 |
| Surfactant | Cremophor EL (v/v%) | 0.66 | 0.47 | - | - | - | - |
| | Cremophor ELP (v/v%) | - | - | 0.81 | 0.27 | 0.81 | 0.27 |
| Buffering agent | Sodium hydrogen phosphate hydrate (w/v%) | 2.72 | 2.72 | 2.72 | 2.72 | 2.72 | 2.72 |
| | Sodium dihydrogen phosphate hydrate (w/v%) | 0.84 | 0.84 | 0.84 | 0.84 | 0.84 | 0.84 |
| | Distilled water (ml) | 100 | 100 | 100 | 100 | 100 | 100 |

**<Table 5>**

| | Component | Example | | | | | |
|---|---|---|---|---|---|---|---|
| | | 25 | 26 | 27 | 28 | 29 | 30 |
| Active ingredient | Cyclosporine (w/v%) | 0.05 | 0.05 | 0.05 | 0.05 | 0.20 | 0.20 |
| Oil | Labrafac PG (v/v%) | - | - | 0.28 | 0.47 | - | - |
| | Lauroglycol FCC (v/v%) | 0.10 | 0.70 | - | - | 0.10 | 0.70 |
| | Lauroglycol 90 (v/v%) | 0.09 | 0.03 | - | - | - | - |
| | Maisine 35-1 (v/v%) | - | - | - | - | 0.09 | 0.03 |
| Surfactant | Labrafil M 1944CS (v/v%) | - | - | 0.06 | 0.06 | - | - |
| | Cremophor ELP (v/v%) | 0.81 | 0.27 | - | - | 0.81 | 0.27 |
| | Cremophor RH 40 (v/v%) | - | - | 0.66 | 0.47 | - | - |
| Buffering agent | Sodium hydrogen phosphate hydrate (w/v%) | 2.72 | 2.72 | 2.72 | 2.72 | 2.72 | 2.72 |
| | Sodium dihydrogen phosphate hydrate (w/v%) | 0.84 | 0.84 | 0.84 | 0.84 | 0.84 | 0.84 |
| | Distilled water (ml) | 100 | 100 | 100 | 100 | 100 | 100 |

**<Table 6>**

| | Component | Example | | | | | |
|---|---|---|---|---|---|---|---|
| | | 31 | 32 | 33 | 34 | 35 | 36 |
| Active ingredient | Cyclosporine (w/v%) | 0.05 | 0.05 | 0.05 | 0.05 | 0.05 | 0.05 |
| Oil | Lauroglycol FCC (v/v%) | 0.50 | 3.50 | - | - | - | - |
| | Lauroglycol 90 (v/v%) | 0.45 | 0.15 | - | - | 0.05 | 0.10 |
| | Plurol oleique CC497 (v/v%) | - | - | 0.05 | 0.10 | - | - |
| Surfactant | Cremophor ELP (v/v%) | 4.05 | 1.35 | - | - | - | - |
| | Solutol HP 15 (v/v%) | - | - | 0.86 | 0.81 | 0.86 | 0.81 |
| | Polysorbate 80 (v/v%) | - | - | 0.10 | 0.09 | 0.10 | 0.09 |
| Buffering agent | Sodium hydrogen phosphate hydrate (w/v%) | 2.72 | 2.72 | 2.72 | 2.72 | 2.72 | 2.72 |
| | Sodium dihydrogen phosphate hydrate (w/v%) | 0.84 | 0.84 | 0.84 | 0.84 | 0.84 | 0.84 |
| | Distilled water (ml) | 100 | 100 | 100 | 100 | 100 | 100 |

**<Table 7>**

| | Component | Example | | | | | |
|---|---|---|---|---|---|---|---|
| | | 37 | 38 | 39 | 40 | 41 | 42 |
| Active ingredient | Cyclosporine (w/v%) | 0.05 | 0.05 | 0.05 | 0.05 | 0.05 | 0.05 |
| Oil | Labrafac PG (v/v%) | - | - | - | - | 0.10 | - |
| | Lauroglycol FCC (v/v%) | - | 0.05 | - | - | - | - |
| | Lauroglycol 90 (v/v%) | 0.05 | - | - | - | - | 0.05 |
| | Maisine 35-1 (v/v%) | - | - | 0.05 | 0.10 | - | - |
| Surfactant | Labrasol (v/v%) | 0.50 | 0.50 | - | - | - | - |
| | Cremophor EL (v/v%) | 0.45 | 0.45 | - | - | - | - |
| | Cremophor ELP (v/v%) | - | - | - | - | 0.80 | 0.86 |
| | Solutol HP 15 (v/v%) | - | - | 0.86 | 0.81 | - | - |
| | Polysorbate 80 (v/v%) | - | - | 0.10 | 0.09 | 0.10 | 0.10 |
| Buffering agent | Sodium hydrogen phosphate hydrate (w/v%) | 2.72 | 2.72 | 2.72 | 2.72 | 2.72 | 2.72 |
| | Sodium dihydrogen phosphate hydrate (w/v%) | 0.84 | 0.84 | 0.84 | 0.84 | 0.84 | 0.84 |
| | Distilled water(ml) | 100 | 100 | 100 | 100 | 100 | 100 |

**<Table 8>**

| | Component | Example | | | | | |
|---|---|---|---|---|---|---|---|
| | | 43 | 44 | 45 | 46 | 47 | 48 |
| Active | Cyclosporine (w/v%) | 0.05 | 0.05 | 0.05 | 0.05 | 0.05 | 0.05 |
| ingredient | | | | | | | |
| Oil | Labrafac PG (v/v%) | 0.05 | 0.14 | 0.08 | 0.08 | 0.07 | 0.05 |
| | Labrafac Lipophile WL 1349 (v/v%) | 0.37 | 0.53 | 0.53 | 0.35 | 0.30 | 0.80 |
| Surfactant | Cremophor ELP (v/v%) | 1.15 | 1.32 | 0.96 | 1.15 | 0.63 | 1.00 |
| Buffering agent | Sodium hydrogen phosphate hydrate (w/v%) | 2.72 | 2.72 | 2.72 | 2.72 | 2.72 | 2.72 |
| | Sodium dihydrogen phosphate hydrate (w/v%) | 0.84 | 0.84 | 0.84 | 0.84 | 0.84 | 0.84 |
| | Distilled water (ml) | 100 | 100 | 100 | 100 | 100 | 100 |

### Experimental Example 1: Measurement of particle size distribution and average particle size

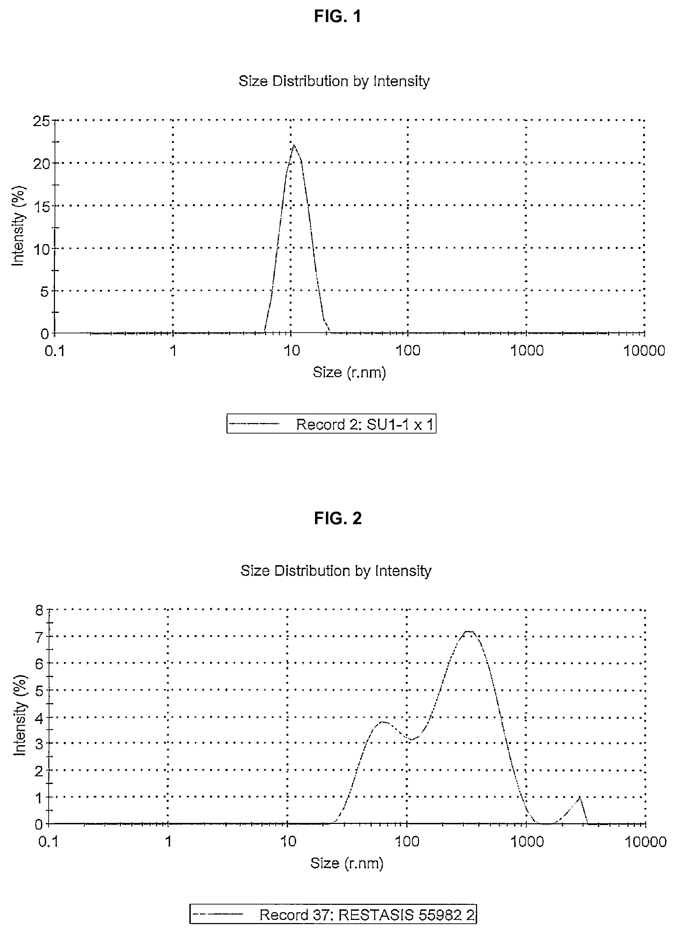

The particle size distributions and the average particle sizes of the cyclosporin A-containing eye drop formulation [Restasis^{™} (Allergan Incorporated)] and the eye drop solutions prepared according to the present disclosure (the eye drop solutions prepared in Examples 1, 5, 7, 12, 31, and 39) were measured. The particle sizes were measured with Zetasizer (Malvern Instruments, England). 2 ml of each sample (eye drop solution) was injected into the Zetasizer without dilution; and then the particle size thereof was measured according to a light scattering method. The particle size distributions of the eye drop solution of Example 1 and Restasis^{™} were shown in FIG. 1 and FIG. 2, respectively. The average particle sizes of the eye drop solutions prepared in Examples 1, 5, 7, 12, 31, and 39 are presented in the following table 9.

**<Table 9>**

| | Example 1 | Example 5 | Example 7 | Example 12 | Example 31 | Example 39 |
|---|---|---|---|---|---|---|
| Average particle size (nm) | 11.20 | 40.22 | 11.20 | 61.22 | 44.71 | 69.58 |

As shown in FIG. 1 and FIG. 2, the eye drop solution prepared according to the present disclosure showed particle size distribution of 11.2 to 69.58 nm, while Restasis^{™} showed broad particle size distribution of 0.067 µm to 2.489 µm. And also, it can be seen that, since the eye drop solutions prepared according to the present disclosure show a very small particle size, it is possible to carry out sterile filtration thereof with a 0.22 µm filter.

### Experimental Example 2: Measurement of transparency and appearance

The transparencies and the appearances of Restasis^{™} (Allergan Incorporated) and the eye drop solutions prepared according to the present disclosure (the eye drop solutions prepared in Examples 1, 5, 7, 12, 31, and 39) were measured. The transparencies were measured with a UV-Vis spectrophotometer (Shimazdu, Japan). 2 ml of each sample (eye drop solution) was injected into the UV-Vis spectrophotometer without dilution; and then each transparency at UV 650 nm through scanning at 700 to 200 nm was measured. The appearances were observed by naked eyes. The results thereof are presented in the following table 10.

**<Table 10>**

| | Restasis™ | Example | | | | | |
|---|---|---|---|---|---|---|---|
| | | 1 | 5 | 7 | 12 | 31 | 39 |
| Transparency at UV 650 nm | 0.3% | 99.0% | 99.0% | 98.2% | 85.2% | 90.6% | 95.3% |
| Appearance | Opaque | Transparent | Transparent | Transparent | Semi-transparent | Semi-transparent | Transparent |

As shown in the table 10, the eye drop solutions prepared according to the present disclosure showed excellent transparencies and transparent/semi-transparent appearances. Therefore, the eye drop solutions prepared according to the present disclosure can minimize blurred vision etc. upon applying to patients' eyes, thereby providing excellent drug compliance.

### Experimental Example 3: Stability test

### (1) Measurement of transparency change

The eye drop solutions prepared according to the present disclosure (the eye drop solutions prepared in Examples 1, 5, 7, 12, 31, and 39) were kept at 50°C for 4 months; and then the transparencies and appearances thereof were measured according to the same methods as in Experimental Example 2. The results thereof are presented in the following table 11.

**<Table 11 >**

| | Example | | | | | |
|---|---|---|---|---|---|---|
| | 1 | 5 | 7 | 12 | 31 | 39 |
| Transparency at UV 650 nm (Initial) | 99.0% | 99.0% | 98.2% | 85.2% | 90.6% | 95.3% |
| Transparency at UV 650 nm (after 4 months) | 98.9% | 99.2% | 97.9% | 83.7% | 90.4% | 94.5% |
| Appearance (after 4 months) | Transparent | Transparent | Transparent | Semi-transparent | Semi-transparent | Transparent |

### (2) Measurement of content change

The eye drop solutions prepared according to the present disclosure (the eye drop solutions prepared in Examples 1, 5, 7, 12, 31, and 39) were kept under the conditions of 40±2 °C and relative humidity 25% for 6 months; and then each content change was measured. The content change was analyzed with HPLC (high performance liquid chromatography) under the following conditions. The results thereof are presented in the following table 12.

### <HPLC condition>

- Wavelength: 210 nm
- Column: a C8 column
- Mobile phase: tetrahydrofuran : 0.03M phosphoric acid solution = 4:6 (v/v)
- Flow rate: 1.0 mL/min

**<Table 12>**

| | Example 1 | Example 5 | Example 7 | Example 12 | Example 31 | Example 39 |
|---|---|---|---|---|---|---|
| Content (initial) | 100.9% | 100.7% | 99.8% | 101.5% | 99.7% | 102.3% |
| Content (after 6 months) | 98.4% | 97.5% | 97.1% | 99.7% | 96.6% | 99.8% |

From the results of the table 11 and the table 12, it can be seen that the eye drop solution in a nanoemulsion form according to the present disclosure forms a thermodynamically stable system, thereby showing excellent physical and chemical stability.

### (3) Long-term stability test

The composition of Examples 1, 5, 7, and 39 were kept at 25±2 °C and 40±2 °C for 15 months; and then the appearances and the presence of precipitation were measured. And also, the composition of Examples 43, 44, 45, 46, 47, and 48 were kept at 25±2 °C and 40±2 °C for 10 months; and then the appearances and the presence of precipitation were measured. The results thereof are presented in the following tables 13 and 14.

**<Table 13>**

| Storage condition (15 months) | | Example | | | |
|---|---|---|---|---|---|
| | | 1 | 5 | 7 | 39 |
| 25±2°C | Appearance | Transparent | Transparent | Transparent | Transparent |
| | Precipitation | No | No | No | No |
| 40±2°C | Appearance | Transparent | Transparent | Transparent | Transparent |
| | Precipitation | No | No | No | No |

**<Table 14>**

| Storage condition (10 months) | | Example | | | | | |
|---|---|---|---|---|---|---|---|
| | | 43 | 44 | 45 | 46 | 47 | 48 |
| 25±2°C | Appearance | Transparent | Transparent | Transparent | Transparent | Transparent | Transparent |
| | Precipitation | No | No | No | No | No | No |
| 40±2°C | Appearance | Transparent | Transparent | Transparent | Transparent | Transparent | Transparent |
| | Precipitation | No | No | No | No | No | No |

From the results of the table 13 and the table 14, it can be seen that the eye drop solutions prepared according to the present disclosure do not show any appearance change and precipitation both under the room temperature-storage condition and under the accelerated storage condition, for long duration.

### Experimental Example 4: Eye irritancy test

Eye irritancy tests were performed on the compositions of the present disclosure. Each composition (about 30 µL, 1 drop) was applied to one eye of 28 healthy adult volunteers; and then the eye irritancy thereof was evaluated, based on the criteria of the table 15, according to the Draize test (skin irritation scores) method. Restasis^{™} was used as a positive control.

**<Table 15>**

| Score | Irritancy |
|---|---|
| 0 | Irritant / no unpleasantness |
| 1 | A little irritant / unpleasantness felt |
| 2 | Irritant / unpleasantness felt (less than 5 minutes) |
| 3 | Irritant / unpleasantness felt (more than 5 minutes) |
| 4 | Strongly irritant (shedding tears) / unpleasantness felt |

As a result of the eye irritancy tests in the above, most of compositions showed more than 50 of the sum total score, i.e., relatively higher eye irritancy, in comparison with the positive control (Restasis^{™}). However, in case of the composition of Example 43, the eye irritancy was remarkably reduced, in comparison with the positive control. Although there is a slight difference in the composition between the composition of Example 43 and the composition of Example 48, it is very interesting that the composition of Example 43 significantly reduces the eye irritancy. The results of the evaluation of eye irritancy are presented in the following table 16.

**<Table 16>**

| Volunteer | Test composition | | | Volunteer | Test composition | | |
|---|---|---|---|---|---|---|---|
| | Positive control (Restasis™) | Composition of Example 43 | Composition of Example 48 | | Positive control (Restasis™) | Composition of Example 43 | Composition of Example 48 |
| 1 | 1 | 1 | 1 | 15 | 0 | 1 | 1 |
| 2 | 2 | 1 | 1 | 16 | 3 | 3 | 2 |
| 3 | 3 | 1 | 4 | 17 | 1 | 1 | 1 |
| 4 | 2 | 1 | 1 | 18 | 1 | 1 | 1 |
| 5 | 3 | 1 | 4 | 19 | 0 | 1 | 1 |
| 6 | 1 | 0 | 1 | 20 | 1 | 0 | 1 |
| 7 | 2 | 3 | 3 | 21 | 2 | 1 | 2 |
| 8 | 2 | 2 | 2 | 22 | 0 | 0 | 1 |
| 9 | 0 | 2 | 2 | 23 | 4 | 2 | 2 |
| 10 | 2 | 1 | 1 | 24 | 0 | 1 | 1 |
| 11 | 1 | 1 | 1 | 25 | 1 | 1 | 1 |
| 12 | 0 | 1 | 1 | 26 | 1 | 1 | 2 |
| 13 | 2 | 3 | 4 | 27 | 3 | 2 | 3 |
| 14 | 2 | 2 | 2 | 28 | 3 | 2 | 2 |
| Sum total score | | | | | 43 | 37 | 49 |
| Average | | | | | 1.54 | 1.32 | 1.75 |

## Claims

1. An ophthalmic composition in a form of nanoemulsion having a particle size of not more than 200 nm, which consists of cyclosporin; propylene glycol dicaprylocaprate and medium chain (C₈ to C₁₀) triglycerides as an oil; polyoxyl 35 hydrogenated castor oil as a surfactant; and sodium hydrogen phosphate or its hydrate, sodium dihydrogen phosphate or its hydrate, or a mixture thereof as a buffering agent; in an aqueous medium.

2. The ophthalmic composition according to claim 1, wherein the oil is present in an amount ranging from 0.05 v/v% to 0.80 v/v%, based on the total composition.

3. The ophthalmic composition according to claim 1, wherein the surfactant is present in an amount ranging from 0.63 v/v% to 1.32 v/v%, based on the total composition.

4. The ophthalmic composition in a form of nanoemulsion having a particle size of not more than 200 nm according to claim 1, which comprises 0.05 w/v% of cyclosporin; 0.05 v/v% of propylene glycol dicaprylocaprate; 0.37 v/v% of medium chain (C₈ to C₁₀) triglycerides; and 1.15 v/v% of polyoxyl 35 hydrogenated castor oil in an aqueous medium.

5. An ophthalmic composition according to claim 1 which consists of 0.05 w/v% of cyclosporin; 0.05 v/v% of propylene glycol dicaprylocaprate; 0.37 v/v% of medium chain (C₈ to C₁₀) triglycerides; 1.15 v/v% of polyoxyl 35 hydrogenated castor oil; 2.72 w/v% of sodium hydrogen phosphate or its hydrate; and 0.84 w/v% of sodium dihydrogen phosphate or its hydrate in an aqueous medium.

## Patentansprüche

1. Ophthalmische Zusammensetzung in einer Nanoemulsionsform mit einer Partikelgröße von nicht mehr als 200 nm, die aus Cyclosporin; Propylenglycoldicaprylocaprat und mittelkettigen (C₈ bis C₁₀) Triglyceriden als ein Öl; Polyoxyl-35-hydriertem-Ricinusöl als ein Tensid; und Natriumhydrogenphosphat oder seinem Hydrat, Natriumdihydrogenphosphat oder seinem Hydrat, oder einem Gemisch davon als ein Puffermittel; in einem wässrigen Medium besteht.

2. Ophthalmische Zusammensetzung nach Anspruch 1, wobei das Öl in einer Menge im Bereich von 0,05 % (v/v) bis 0,80 % (v/v), bezogen auf die Gesamtzusammensetzung vorliegt.

3. Ophthalmische Zusammensetzung nach Anspruch 1, wobei das Tensid in einer Menge im Bereich von 0,63 % (v/v) bis 1,32 % (v/v), bezogen auf die Gesamtzusammensetzung vorliegt.

4. Ophthalmische Zusammensetzung in einer Nanoemulsionsform mit einer Partikelgröße von nicht mehr als 200 nm nach Anspruch 1, die 0,05 % (w/v) Cyclosporin; 0,05 % (v/v) Propylenglycoldicaprylocaprat; 0,37 % (v/v) mittelkettige (C₈ bis C₁₀) Triglyceride; und 1,15 % (v/v) Polyoxyl-35-hydriertes-Ricinusöl in einem wässrigen Medium enthält.

5. Ophthalmische Zusammensetzung nach Anspruch 1, die aus 0,05 % (w/v) Cyclosporin; 0,05 % (v/v) Propylenglycoldicaprylocaprat; 0,37 % (v/v) mittelkettigen (C₈ bis C₁₀) Triglyceriden; 1,15 % (v/v) Polyoxyl-35-hydriertem-Ricinusöl; 2,72 % (w/v) Natriumhydrogenphosphat oder seinem Hydrat; und 0,84 % (w/v) Natriumdihydrogenphosphat oder seinem Hydrat in einem wässrigen Medium besteht.

## Revendications

1. Composition ophtalmique sous forme de nanoémulsion ayant une dimension de particules égale ou inférieure à 200 nm, qui comprend de la cyclosporine ; du dicaprylocaprate de propylène glycol et des triglycérides à chaîne moyenne (C₈ à C₁₀) en tant qu'huile ; de l'huile de ricin hydrogénée et éthoxylée contenant 35 motifs en tant qu'agent tensioactif ; et de l'hydrogénophosphate de sodium ou son hydrate, du dihydrogénophosphate de sodium ou son hydrate, ou leur mélange en tant que tampon ; dans un milieu aqueux.

2. Composition ophtalmique selon la revendication 1, dans laquelle l'huile est présente à raison d'une quantité comprise entre 0,05 volume/volume pour cent et 0,80 volume/volume pour cent, par rapport à la composition totale.

3. Composition ophtalmique selon la revendication 1, dans laquelle l'agent tensioactif est présent à raison d'une quantité comprise entre 0,63 volume/volume pour cent à 1,32 volume/volume pour cent, par rapport à la composition totale.

4. Composition ophtalmique sous forme de nanoémulsion ayant une dimension de particules égale ou inférieure à 200 nm selon la revendication 1, qui comprend 0,05 poids/volume pour cent de cyclosporine ; 0,05 volume/volume pour cent de dicaprylocaprate de propylène glycol ; 0,37 volume/volume pour cent de triglycérides à chaîne moyenne (C₈ à C₁₀) ; et 1,15 volume/volume pour cent d'huile de ricin hydrogénée et éthoxylée contenant 35 motifs dans un milieu aqueux.

5. Composition ophtalmique selon la revendication 1, qui comprend 0,05 poids/volume pour cent de cyclosporine ; 0,05 volume/volume pour cent de dicaprylocaprate de propylène glycol ; 0,37 volume/volume pour cent de triglycérides à chaîne moyenne (C₈ à C₁₀) ; 1,15 volume/volume pour cent d'huile de ricin hydrogénée et éthoxylée contenant 35 motifs ; 2,72 poids/volume pour cent d'hydrogénophosphate de sodium ou son hydrate ; et 0,84 poids/volume pour cent de dihydrogénophosphate de sodium ou son hydrate dans un milieu aqueux.
